# EUROPEAN PATENT SPECIFICATION

(11) **EP 3 986 379 B1**
(45) Date of publication and mention of the grant of the patent: **23.10.2024**
(21) Application number: 19735158.8
(22) Date of filing: 19.06.2019
(51) Int. Cl.: A61K 9/16, A61K 9/127, A61K 31/44, A61K 31/704

(54) **BIODEGRADABLE DRUG-ELUTING EMBOLIC PARTICLES FOR DELIVERY OF THERAPEUTIC AGENTS**
BIOLOGISCH ABBAUBARE EMBOLISCHE PARTIKEL ZUR VERABREICHUNG THERAPEUTISCHER AGENZIEN
PARTICULES BIODEGRADABLES EMBOLIQUES POUR L' ADMINISTRATION D' AGENTS THERAPEUTIQUES

(43) Date of publication of application: 27.04.2022
(73) Proprietor: Becton, Dickinson and Company, Franklin Lakes, NJ 07417 (US)
(72) Inventor: AFONINA, Elena, Franklin Lakes, New Jersey 07417 (US); DO, Hiep, Franklin Lakes, New Jersey 07417 (US); GOEL, Emily, Franklin Lakes, New Jersey 07417 (US); GURYEV, Oleg, Franklin Lakes, New Jersey 07417 (US); LECY, Cyal, Franklin Lakes, New Jersey 07417 (US); MEHRPOUYAN, Majid, Franklin Lakes, New Jersey 07417 (US); RANDALL, Michael, Franklin Lakes, New Jersey 07417 (US); SHARKEY, Marybeth, Franklin Lakes, New Jersey 07417 (US); WANG, Jeffrey, Franklin Lakes, New Jersey 07417 (US)
(74) Representative: Hoffmann Eitle
(86) International application number: PCT/US2019/037984
(87) International publication number: WO 2020/256722

(56) References cited:
- EP-A2- 2 891 485
- WO-A1-2018/064150
- WO-A1-2018/218208
- WO-A2-2009/073193

## Description

### TECHNICAL FIELD

This disclosure relates generally to particles for chemoembolization and, more particularly, to biodegradable drug-eluting particles containing therapeutic agents for chemoembolization.

### BACKGROUND

Embolization therapy is a minimally invasive surgery performed by interventional radiologists. Typical treatments may include entering the vasculature via a minor incision, such as in the arm or leg, and gaining access to the treatment site by use of guidewires and catheters, optionally aided by imaging techniques such as fluoroscopy. The embolic agent at the treatment site embolizes the vessel, blocking off the flow of blood to tumors downstream from the treatment site and resulting in necrosis and/or shrinkage of the tumors.

The embolic agent of choice for embolization therapy depends on the desired clinical outcome, as well as the inherent properties of the embolic agent. Embolic agents used clinically suffer common drawbacks. First, the embolic agents are provided without preloaded drug, meaning that the physician must order a pharmacy to load the drug into the embolic agent well in advance of surgery, typically at least 24 hours before the surgery. Second, the embolic agents have a suboptimal pharmacokinetic release profile that stops releasing drug into the target treatment site after only a few days after implantation, typically within about 3 days after implantation.

Among the diseases for which embolization therapy may be effective is hepatocellular carcinoma (HCC). Hepatocellular carcinoma accounts for 80% to 90% of all primary liver cancers. The mortality of hepatocellular carcinoma is high, and treatment options are limited. Patients with cirrhosis exhibit a high probability of developing hepatocellular carcinoma.

Sorafenib (co-developed and co-marketed by Bayer and Onyx Pharmaceuticals as Nexavar) is an orally administered kinase inhibitor drug approved for the treatment of primary kidney cancer (advanced renal cell carcinoma), radioactive iodine resistant advanced thyroid carcinoma, and advanced primary liver cancer such as hepatocellular carcinoma. With the exception of sorafenib tosylate (the toluene salt of sorafenib), no targeted agent is currently approved for the treatment of hepatocellular carcinoma.

Cytotoxic agent doxorubicin exhibits a broad spectrum of antitumor activity. When administrated via the hepatic artery, doxorubicin exhibits antitumor effects and partial response in hepatocellular carcinoma patients. An exploratory analysis of a randomized phase II study in hepatocellular carcinoma comparing doxorubicin alone to doxorubicin plus sorafenib showed a significant improvement in overall survival favoring the combination.

Ongoing needs exist for embolic agents and embolization therapy methods that enable greater efficiency in preparation of the embolic agents and that also provide sustained, longerterm delivery of therapeutic agent to the embolization site. Furthermore, ongoing needs exist for embolic agents that are capable of delivering multiple therapeutic agents, including but not limited to the combination of sorafenib and doxorubicin, in a controlled manner as an alternative to oral administration. WO 2018/218208 discloses a chemoembolization agent for treatment of vascularized solid tumor cancer, the chemoembolization agent comprising an embolizing particle or microsphere, an encapsulating agent, the encapsulating agent affixed to the embolizing particle or microsphere through ionic or other non-covalent interactions, and the encapsulating agent being a liposome, and a therapeutic agent contained within the encapsulating agent.

### SUMMARY

Against the above background, the present invention is directed to drug-loaded microbead compositions including a plurality of individual microbeads of a biodegradable material, a plurality of individual vesicular agents, a first therapeutic agent, and a second therapeutic agent different from the first therapeutic agent. The vesicular agents comprise liposomes or ethosomes The first therapeutic agent is associated with the individual vesicular agents. For example, the first therapeutic agent may be contained within the individual vesicular agents or may be associated with the individual vesicular agents by ionic or non-covalent interaction. The second therapeutic agent is different from the first therapeutic agent, is contained within the individual microbeads or associated with the individual microbeads through ionic or non-covalent interaction, and may or may not be associated with the individual vesicular agents within the individual microbeads.

Example embodiments disclosed herein are directed to methods for preparing the drug-loaded microbead compositions, to embolization compositions prepared from the drug-loaded microbead compositions, to methods for preparing the embolization compositions, and to methods for treating a disease using the embolization compositions.

Further embodiments of this disclosure are directed to drug-loaded biodegradable microbead compositions. The drug-loaded biodegradable microbead compositions may include microbeads of a biodegradable material and at least one therapeutic agent embedded in a matrix of the biodegradable material, encapsulated by a matrix of the biodegradable material, disposed within a porous structure formed by the matrix of the biodegradable material, or ionically or non-covalently associated with a matrix of the biodegradable material. In such embodiments of drug-loaded biodegradable microbead compositions, the microbeads of the biodegradable material may contain, but need not necessarily contain, vesicular agents such as liposomes or ethosomes.

Additional features and advantages of the embodiments described herein will be set forth in the detailed description which follows, and in part will be readily apparent to those skilled in the art from that description or recognized by practicing the embodiments described herein, including the detailed description which follows, the claims, as well as the appended drawings.

### DETAILED DESCRIPTION

Reference will now be made in detail to embodiments of drug-loaded microbead compositions including microbeads or a biodegradable material loaded with at least two therapeutic agents. The microbead compositions according to embodiments include individual microbeads that contain a plurality of individual liposomes or ethosomes. At least one of the therapeutic agents is contained within the liposomes or ethosomes, either incorporated within a lipid bilayer or surrounded by a lipid bilayer. Another of the therapeutic agents may be contained within the individual vesicular agents or may be incorporated within a matrix formed by the biodegradable material of the microbeads but outside the individual vesicular agents. Further embodiments of this disclosure are directed to methods for preparing the drug-loaded microbead compositions, to embolization compositions prepared from the drug-loaded microbead compositions, to methods for preparing the embolization compositions, and to methods for treating a disease using the embolization compositions. The drug-loaded microbead compositions include microbeads into which a therapeutic agent has been pre-loaded. When included in an embolization composition and provided to a subject as part of an embolization therapy, the embolization compositions may provide a continuous release of the drug into the target treatment site for at least 7 days, at least 14 days, at least 21 days, or at least 30 days after implantation.

According to embodiments, drug-loaded microbead compositions include plurality of individual microbeads of a biodegradable material, a plurality of individual vesicular agents, a first therapeutic agent, and a second therapeutic agent different from the first therapeutic agent. In some embodiments, the plurality of individual vesicular agents may be contained within the individual microbeads, such as within pores of the individual microbeads. In some embodiments, the plurality of individual vesicular agents may be associated with the biodegradable material of the individual microbeads through ionic or non-covalent interaction, either entirely encompassed with a matrix of the biodegradable material or attached to the biodegradable material such as on an outer surface of the individual microbeads, for example. The vesicular agents include at least one lipid bilayer surrounding a vesicular core. The first therapeutic agent is associated with the individual vesicular agents. For example, the first therapeutic agent may be contained within the individual vesicular agents or may be associated with the individual vesicular agents by ionic or non-covalent interaction. The second therapeutic agent is different from the first therapeutic agent and is contained within the individual microbeads or associated with the individual microbeads through ionic or non-covalent interaction.

With respect to the vesicular agents and the therapeutic agents, the term "associated with" includes non-covalent interactions such as ionic bonds, van der Waals interactions, hydrogen bonding, pi-pi stacking, dipole-dipole interactions, dipole-quadrupole interactions, quadrupole-quadrupole interactions, multipole-multipole interactions, or combinations thereof. Ionic interactions or ionic bonding include a type of chemical bonding that involves the electrostatic attraction between oppositely charged ions such as anions or cations. In some examples, a therapeutic agent may include atoms or groups capable of forming ions or may have one or more natural dipoles. In such examples, the ions or dipoles may have particular affinity toward an oppositely charged ion or dipole naturally present in the vesicular agent or the biodegradable material. This affinity, in turn, results in a reversible chemical attraction or combination between the therapeutic agent and the vesicular agent or the biodegradable material.

The individual microbeads of the drug-loaded microbead compositions are microbeads of a biodegradable material. The biodegradable material or the individual microbeads forms a bead matrix that may include pores and may have various shapes or sizes. In some embodiments, the microbeads are spherical or ovoid. In some embodiments, the biodegradable material is a biodegradable polymer or a lipid. Examples of biodegradable polymers include, without limitation, poly(lactide-co-glycolide) (PLGA), polylactide(PLA), polyglycolide (PGA), polycaprolactone (PCL), polyhydroxyalkanoates, poly-R-3-hydroxybutyrate (poly3HB), poly-R-3-hydroxy-butyrate-co-R-3-hydroxyvalerate (poly(3HB-co-3HV)), poly-R-3-hydroxybutyrate-co-4-hydroxybutyrate (poly(3HB-co-4HB)), poly-R-3-hydroxyoctanoate-co-R-3-hydroxy-hexanoate (poly(3HO-co-3HH)), poly-3-hydroxypropionate (poly(3HP)), poly-4-hydroxy-butyrate (poly(4HB)), poly-5-hydroxybutyrate (poly(5HB)), poly-6-hydroxy-butyrate (poly(6HB)), poly(propylene fumarate), poly(butylene succinate), poly(p-dioxanone, polyacetals, poly(ortho esters), polycarbonates, chitosan, hydroxybutyric acids, polyanhydrides and polyesters, polyphosphazenes, polyphosphoesters, lipodisq, celluloses, modified celluloses, proteins and poly(amino acids), polyethers, and co-polymers of the foregoing. Examples of lipids include, without limitation, tricaprin, trilaurin, trimyristin, tripalmitin, tristearin, hydrogenated coco-glycerides, glyceryl monostearate, glyceryl behenate, glyceryl palmitostearate, glyceryl caprate, cetyl palmitate, stearic acid, palmitic acid, decanoic acid, behenic acid, beeswax, carnauba wax, cacao butter, and combinations thereof. In example embodiments, the drug-loaded microbead compositions include individual microbeads are beads of a biodegradable cellulose or a biodegradable modified cellulose such as cellulose acetate butyrate.

The biodegradable polymer materials of the drug-loaded microbead composition may include derivatives of any of the foregoing materials, or may include combinations of any of the foregoing materials or their derivatives. For example, the drug-loaded microbead compositions may include a combination of multiple biodegradable polymer materials, in which each individual microbead is made of a single type of polymer, and the drug-loaded microbead composition includes microbeads of multiple polymer types. Alternatively, the drug-loaded microbead compositions may include a combination of multiple biodegradable polymer materials, in which individual microbeads of the composition are composed of multiple types of polymer.

In various embodiments, the biodegradable material of the drug-loaded microbead composition may include a solid lipid such as tricaprin, trilaurin, trimyristin, tripalmitin, tristearin, hydrogenated coco-glycerides, glyceryl monostearate, glyceryl behenate, glyceryl palmitostearate, glyceryl caprate, cetyl palmitate, stearic acid, palmitic acid, decanoic acid, behenic acid, beeswax, carnauba wax, and cacao butter. The biodegradable materials of the drug-loaded microbead composition may include derivatives of any of the foregoing solid lipids, or may include combinations of any of the foregoing solid lipids or their derivatives. For example, the drug-loaded microbead compositions may include a combination of multiple solid lipids, in which each individual microbead is made of a single type of solid lipid, and the drug-loaded microbead composition includes microbeads of multiple solid lipids. Alternatively, the drug-loaded microbead compositions may include a combination of multiple solid lipids, in which individual microbeads of the composition are composed of multiple types of solid lipids.

In the drug-loaded microbead compositions according to the invention, the vesicular agents include liposomes and ethosomes. Liposomes and ethosomes are particles having at least one amphipathic, spherical or nearly spherical bilayer formed by van der Waals interactions between a plurality of hydrophobic moieties each capped by a polar head group and arranged in an alternating manner such that a polar head group of one hydrophobic moiety projects outwardly to an external aqueous environment, while an adjacent hydrophobic moiety projects its polar head group inwardly. Liposomes can be classified according to their lamellarity (uni- and multilamellar vesicles,), size (small, intermediate, or large), and charge (anionic, cationic and neutral) of the polar head groups. Liposome particles typically have diameters from about 0.025 µm to about 2.5 µm, in which the hydrophobic moieties are linear or lightly branched saturated hydrocarbons. Ethosomes are distinguished from liposomes in that the vesicular core of ethosomes includes aqueous ethanol solution. In embodiments, a therapeutic agent may be dissolved within the aqueous ethanol solution of ethosomes.

The liposomes or ethosomes, for example, include at least one lipid bilayer. The lipid bilayer may include a phospholipid. Examples of phospholipids include, without limitation, poly(2-methacryloyloxyethyl phosphorylcholine) (PMPC), soybean phosphatidylcholine (SPC), hydrogenated soybean phosphatidylcholine (HSPC), egg sphingomyelin (ESM), egg phosphatidylcholine (EPC), dimyristoyl phosphatidylcholine (DMPC), dipalmitoyl phosphatidylcholine (DPPC), dioleoyl phosphatidylcholine (DOPC), distearoyl phosphatidylcholine (DSPC), dimyristoyl phosphatidylglycerol (DMPG), dipalmitoyl phosphatidylglycerol (DPPG), dioleoyl phosphatidylglycerol (DOPG), distearoyl phosphatidylglycerol (DSPG), dimyristoyl phosphatidylethanolamine (DMPE), dipalmitoyl phosphatidylethanolamine (DPPE), dioleoyl phosphatidylethanolamine (DOPE), dimyristoyl phosphatidylserine (DMPS), dipalmitoyl phosphatidylserine (DPPS), dioleoyl phosphatidylserine (DOPS), 1,2-dioleoyl-3-trimethylammonium -propane (DOTAP), 1,2-dilauroyl-sn-glycero-3-phosphocholine (DLPC), 1,2-dimyristoyl-sn-glycero-3-phosphocholine (DMPC), 1,2-dimyristoyl-sn-glycero-3-phosphate (sodium salt) (DMPA·Na), 1,2-dipalmitoyl-sn-glycein-3 -phosphate (sodium salt) (DPPA·Na), 1,2-dioleoyl-sn-glycero-3-phosphate (sodium salt) (DOPA·Na), 1,2-dimyristoyl-sn-glycero-3-phospho-(1'-rac-glycerol) (sodium salt) (DMPG·Na), 1,2-dipalmitoyl-sn-glycero-3-phospho-(1'-rac-glycerol) (sodium salt) (DPPG·Na), 1,2-dioleoyl-sn-glycero-3-phospho-(1'-rac-glycerol) (sodium salt) (DOPG·Na), 1,2-dimyristoyl-sn-glycero-3-phospho-L-serine (sodium salt) (DMPS·Na), 1,2-dipalmitoyl-sn-glycero-3-phospho-L-serine (sodium salt) (DPPS·Na), 1,2-dioleoyl-sn-glycero-3-phaspho-L-serine (sodium salt) (DOPS-Na), 1,2-dioleoyl-sn-glycero-3-phosphoethanolamine-N-(glutaryl) (sodium salt) (DOPE-glutaryl (Na)₂), 1',3'-bis[1,2-dimyristoyl-sn-glycero-3-phospho]-sn-glycerol (ammonium salt) (Tetramyristoyl Cardiolipin (Na)₂), 1,2-distearoyl-sn-glycero-3-phosphoethanolamine-N-[methoxy(polyethylene glycol)-2000] (ammonium salt) (DSPE-mPEG-2000, Na), 1,2-distearoyl-sn-glycero-3-phosphoethanolamine-N-[methoxy (polyethylene glycol)-5000] (ammonium salt) (DSPE-mPEG-5000·Na), 1,2-distearoyl-sn-glycero-3-phosphoethanolamine-N-[maleimide(polyethylene glycol)-2000] (ammonium salt) (DSPE-Maleimide PEG-2000·Na), 1,2-dioleoyl-3-trimethylammonium -propane (chloride salt) (DOTAP-C1), 1,2-dihexanoyl-sn-glycero-3-phosphocholine (DHPC),and mixtures thereof, and salts thereof. In example embodiments, the lipid bilayer of the vesicle agents such as liposomes or ethosomes may be a combination of lipids. In a specific embodiment, the lipid bilayer of the vesicle agents such as liposomes or ethosomes may be a combination of poly(2-methacryloyloxyethyl phosphorylcholine) (PMPC), dioleoyl phosphatidylserine (DOPS), dioleoyl phosphatidylethanolamine (DOPE), and cholesterol.

The individual microbeads of the drug-loaded microbead compositions according to embodiments include at least two therapeutic agents, such as the first therapeutic agent and the second therapeutic agent. The first therapeutic agent is associated with or carried within the individual vesicular agents. For example, the first therapeutic agent may be contained within the individual vesicular agents or may be associated with the individual vesicular agents by ionic or non-covalent interaction. The first therapeutic agent may be within the lipid bilayer of the vesicular agents, within the vesicular core of the vesicular agents, or bound to the outer surface of the lipid bilayer and, therefore, not encapsulated by the vesicular agents.

The second therapeutic agent is different from the first therapeutic agent. The second therapeutic agent is contained within the individual microbeads or associated with the individual microbeads through ionic or non-covalent interaction. In some embodiments, the second therapeutic agent is contained within the individual vesicular agents of the individual microbeads or associated with individual vesicular agents through ionic or non-covalent interaction. For example, in some embodiments, the second therapeutic agent may be within the lipid bilayer of the vesicular agents, within the vesicular core of the vesicular agents, or bound to the outer surface of the lipid bilayer and, therefore, not encapsulated by the vesicular agents. In some embodiments, the second therapeutic agent is not contained within the individual vesicular agents and not ionically or non-covalently associated with the individual vesicular agents but, rather, is associated by ionic or non-covalent interaction with the biodegradable material of the individual microbeads. In such embodiments, the individual microbeads may contain vesicular agents loaded with the first therapeutic agent and molecules of the second therapeutic agent inside or attached to the matrix of biodegradable material that forms the individual microbeads but not inside the vesicular agents.

In some embodiments of the drug-loaded microbead composition the first therapeutic agent is hydrophobic and the second therapeutic agent is hydrophilic. In example embodiments, the first therapeutic agent may be encapsulated within the vesicular core of the individual vesicular agents and the second therapeutic agent may be contained within the lipid bilayer of the individual vesicular agents. In further example embodiments, the first therapeutic agent may be encapsulated within the vesicular core of the individual vesicular agents and the second therapeutic agent is not contained within the individual vesicular agents and is associated by ionic or non-covalent interaction with the biodegradable material of the individual microbeads.

The drug-loaded microbead compositions include at least two therapeutic agents. In embodiments, the first therapeutic agent and the second therapeutic agent are independently chosen from doxorubicin, bevacizumab, bortezomib, imatinib, seliciclib, ceritinib, everolimus, paclitaxel, sorafenib, irinotecan, idarubicin, cisplatin, pharmaceutically acceptable salts and derivatives thereof, and combinations thereof, provided the first therapeutic agent is different from the second therapeutic agent. In some embodiments, the therapeutic agent may be a hydrophilic therapeutic agent or a therapeutic agent that either is water soluble or has at least some solubility in an aqueous solution. In some embodiments, the therapeutic agent may be a chemotherapeutic agent having at least some efficacy for treating a disease such as cancer. In some embodiments, the therapeutic agent may be a chemotherapeutic agent having at least some efficacy for treating a cancer such as hepatocellular carcinoma, liver cancer, prostate cancer, or breast cancer. The therapeutic agent may have one or more chemical moieties or atomic centers having a positive or negative charge or affinity. Examples of specific therapeutic agents include, without limitation, doxorubicin, sorafenib, vandetanib, nivolumab, ipilimumab, regorafenib, irinotecan, epirubicin, pirarubicin, 5-fluorouracil, cisplatin, floxuridine, mitomycin C, derivatives of any of the foregoing, prodrugs of any of the foregoing, therapeutically acceptable salts or crystalline forms of any of the foregoing, or combinations of any of the foregoing. Further examples of suitable therapeutic agents include, without limitation, pirarubicin, mitoxantrone, tepotecan, paclitaxel, carboplatin, pemetrexed, penistatin, pertuzumab, trastuzumab, and docetaxel. Still further examples of suitable therapeutic agents include, without limitation, doxorubicin, bevacizumab, bortezomib, imatinib, seliciclib, ceritinib, everolimus, paclitaxel, sorafenib, irinotecan, idarubicin, cisplatin, and combinations of these drugs.

In an example embodiment of a drug-loaded microbead composition, the individual vesicular agents include liposomes; the first therapeutic agent is contained within the lipid bilayer of the individual vesicular agents; the second therapeutic agent is not contained within the individual vesicular agents and is associated by ionic or non-covalent interaction with the biodegradable material of the individual microbeads; and the biodegradable material is a cellulose. In such embodiments, the liposomes may include a lipid bilayer formed from a mixture of poly(2-methacryloyloxyethyl phosphorylcholine) (PMPC), dioleoyl phosphatidylserine (DOPS), dioleoyl phosphatidylethanolamine (DOPE), and cholesterol, for example.

In a further example embodiment of a drug-loaded microbead composition, the individual vesicular agents include liposomes; the first therapeutic agent is contained within the lipid bilayer of the individual vesicular agents; the second therapeutic agent is encapsulated within the vesicular core of the individual vesicular agents; the first therapeutic agent is sorafenib; the second therapeutic agent is doxorubicin; and the biodegradable material is a cellulose or modified cellulose such as ethyl cellulose or cellulose acetate butyrate. In such embodiments, the liposomes may include a lipid bilayer formed from a mixture of poly(2-methacryloyloxyethyl phosphorylcholine) (PMPC), dioleoyl phosphatidylserine (DOPS), dioleoyl phosphatidylethanolamine (DOPE), and cholesterol, for example.

In a further example embodiment of a drug-loaded microbead composition, the individual vesicular agents include ethosomes; the first therapeutic agent is encapsulated within the vesicular core of the individual vesicular agents; the second therapeutic agent is not contained within the individual vesicular agents and is associated by ionic or non-covalent interaction with the biodegradable material; the first therapeutic agent is sorafenib; the second therapeutic agent is doxorubicin; and the biodegradable material is a cellulose or modified cellulose such as ethyl cellulose or cellulose acetate butyrate. In such embodiments, the liposomes may include a lipid bilayer formed from a mixture of poly(2-methacryloyloxyethyl phosphorylcholine) (PMPC), dioleoyl phosphatidylserine (DOPS), dioleoyl phosphatidylethanolamine (DOPE), and cholesterol, for example.

In the individual microbeads of the drug-loaded microbead composition, the therapeutic agent or a complex of the therapeutic agent may be encapsulated by or embedded within the matrix formed by the biodegradable material. Except when described otherwise, the terms "encapsulated" and "embedded" broadly include embodiments for which the biodegradable material or some portion thereof generally surrounds the therapeutic agent and/or the vesicular agents including a therapeutic agent. For example, "embedded" may include in some embodiments a biodegradable material shell that encapsulates a core that holds the therapeutic agent and/or vesicular agents. In other embodiments, "embedded" may include a structure in which the therapeutic agent or the complex is physically disposed within a matrix, network, or pore structure of a biodegradable material that may or may not have a core within an outer shell. The therapeutic agent itself may not be chemically bonded to the biodegradable material at all or may not be chemically bonded directly to the polymer backbone of the biodegradable material when the biodegradable material is a biodegradable polymer material. The drug-loaded microbead composition may have a water content of less than 1% by weight, based on the total weight of the drug-loaded microbead composition.

In some embodiments, the therapeutic agent of the drug-loaded microbead composition may be embedded within the microbeads of the biodegradable material but not chemically bonded to the biodegradable material. In some embodiments, the therapeutic agent of the drug-loaded microbead composition may be embedded within the microbeads of the biodegradable material and not chemically bonded directly to a polymer backbone of the biodegradable material, yet may be chemically bonded to a functional group of the biodegradable material. As used herein, "not chemically bonded" refers to a lack of covalent chemical bonds between the therapeutic agent and the biodegradable but does not preclude the existence of noncovalent intermolecular interactions such as ionic interactions or a van der Waals interaction between the therapeutic agent and the biodegradable material.

In some embodiments, the vesicular agents form complexes of the therapeutic agent. In the complex, the therapeutic agent may be chemically bonded to the vesicular agents or the lipid bilayer thereof or may be associated with the vesicular agents or the lipid bilayer thereof by a non-covalent means such as encapsulation or a van der Waals interaction. The complex may be embedded within the biodegradable material. When the complex is embedded within the microbeads, the vesicular agents may be chemically bonded to the biodegradable material while the therapeutic agent is not chemically bonded to the biodegradable material. Without intent to be bound by theory, it is believed that when the therapeutic agent is bonded or associated with the vesicular agents but is not chemically bonded to the biodegradable material, the microbeads of the drug-loaded microbead composition are less susceptible to shrinking as a result of replacing water molecules with drug molecules during drug loading. Accordingly, the final size distribution of the drug-loaded microbeads can be controlled more readily by selecting appropriate microbead sizes before the therapeutic agent is loaded.

The drug-loaded microbead compositions according to embodiments may have a very low water content such as less than 1%, or less than 0.1%, or less than 0.05% (500 ppm), or less than 0.02% (200 ppm), or less than 0.01% (100 ppm), or less than 0.005 (50 ppm), or less than 0.002% (20 ppm), or less than 0.001% (10 ppm) by weight water, based on the total weight of the microbeads in the drug-loaded microbead composition. Without intent to be bound by theory, it is believed that a very low water content of the drug-loaded microbead composition increases the shelf-life and long-term stability of the drug-loaded microbead composition. Further, it is believed that water contents significantly greater than 1% by weight (such as 2%, 3%, 5%, or 10%, for example) based on the total weight of the drug-loaded microbead composition, may lead to decomposition or hydrolysis of the therapeutic agent, instability or breaking apart of the water-swellable polymer, or a combination of these, within a few days or even a few hours, such that the composition cannot be used for embolization procedures, even if the drug-loaded microbead composition is rehydrated. It is believed that the shelf-life and long-term stability of compositions having water contents significantly greater than 1% by weight are not sufficiently long to ensure viability of the therapeutic agent over the time period from manufacture of the drug-loaded microbead composition to use of the composition in an embolization procedure. It is believed that selection of the water-swellable polymer material may correlate with the ability for water to be removed from the drug-loaded microbeads by lyophilization or other drying technique or combination of drying techniques in an amount sufficient to prevent decomposition of the therapeutic agent.

A very low water content of the drug-loaded microbead composition, as previously described may be attained by drying techniques that will be described in greater detail subsequently, with respect to methods for preparing the drug-loaded microbead compositions. In this regard, the drug-loaded microbead compositions may be dry or nearly dehydrated compositions of the microbeads containing the embedded therapeutic agent or the embedded complex of the therapeutic agent and the vesicular agents. The drug-loaded microbead compositions may have a powder-like consistency. Accordingly, the drug-loaded microbead compositions may be made suitable for injection into a subject being treated by rehydrating the microbeads of the drug-loaded microbead compositions to form an embolization composition, as will be described in greater detail subsequently. Regardless, the drug-loaded microbead compositions may be provided in such a form that a physician needs to add only an aqueous solution such as water or physiologically buffered saline solution to the drug-loaded microbead composition to prepare the composition for use in an embolization procedure.

The microbeads of the drug-loaded microbead composition may have any shape common to microparticles formed from a hydrogel type water-swellable polymer material. For example, the microparticles may be spherical or substantially spherical, or may have an ovoid shape with oval-shaped or elliptical cross-sections about a longitudinal axis and circular cross-sections about an axis perpendicular to the longitudinal axis.

The drug-loaded microbead composition may include an amount of therapeutic agent per unit volume of microbeads in the composition that is chosen to have a desired therapeutic effect or activity, based on the intended use for the drug-loaded microbead composition and the particular therapeutic agent present in the individual microbeads.

The amount of therapeutic agent in the individual microbeads of the drug-loaded microbead composition can be adjusted through particular techniques involved during drug loading, such as loading time, loading temperature, or concentration of therapeutic agent in a loading solution, for example. The amount of therapeutic agent in the individual microbeads of the drug-loaded microbead composition can be adjusted also through synthetic techniques involved for synthesizing the microbeads themselves, such as through adjusting polymer molecular weights, polymer density, or polymer porosity of the biodegradable material. For example, when doxorubicin is the therapeutic agent, the amount of drug loading in the drug-loaded microbeads can be adjusted with respect to the number of negative charges in the polymer backbone of the water-swellable polymer material. Similarly, when sorafenib is the therapeutic agent, the sorafenib may be embedded within liposomes that are embedded within the microbead structure.

In example embodiments, the individual drug-loaded microbeads of the drug-loaded microbead composition include the biodegradable material, the first and second therapeutic agents, and water. The individual drug-loaded microbeads of the drug-loaded microbead composition may include from about 30% by weight to about 70% by weight, or from about 35% by weight to about 65% by weight, or from about 40% to about 60% by weight, or about 45% by weight to about 55% by weight, or about 50% to about 70% by weight biodegradable material, based on the total weight of the individual drug-loaded microbead. In example embodiments, the individual drug-loaded microbeads of the drug-loaded microbead composition may include from about 1% by weight to about 25% by weight, or from about 1% by weight to about 20% by weight, or from about 1% by weight to about 15% by weight, or from about 2% by weight to about 25% by weight, or from about 5% by weight to about 25% by weight, or from about 10% by weight to about 25% by weight therapeutic agent, based on the total weight of the individual drug-loaded microbead. In example embodiments, the individual drug-loaded microbeads of the drug-loaded microbead composition according to embodiments may have a very low water content such as less than 1% by weight, or less than 0.5% by weight, or less than 0.1% by weight, or less than 0.05% (500 ppm) by weight, or less than 0.02% (200 ppm) by weight, or less than 0.01% (100 ppm) by weight, or less than 0.005 (50 ppm) by weight, or less than 0.002% (20 ppm) by weight, or less than 0.001% (10 ppm) by weight water, based on the total weight of the individual microbeads in the drug-loaded microbead composition.

Having described drug-loaded microbead compositions according to various embodiments, methods for preparing the drug-loaded microbead compositions will now be described.

Example methods for preparing drug-loaded microbead compositions include preparing vesicular agents, adding the vesicular agents to an aqueous solution, preparing an organic solution containing a precursor to a biodegradable material, combining the aqueous solution and the organic solution, agitating the mixture to form microbeads, drying the microbeads, and recovering the microbeads. The methods may further include washing, rinsing, or filtering the microbeads.

The foregoing methods for preparing drug-loaded microbead compositions, regardless of whether the drug-loaded microbeads are prepared including the intermediate step of forming particles of the complexes of therapeutic agent and vesicular agents or without the intermediate step, further include removing water from the drug-loaded microbeads to form a drug-loaded microbead composition having a water content of less than 1% by weight, based on the total weight of the drug-loaded microbead composition. In some embodiments, the removal of the water may include lyophilization or freeze drying, optionally followed by an additional drying step involving temperature variation (heating or cooling), flowing air, vacuum, or a combination of these. The drug-loaded microbeads may have an average synthesized volume upon initial recovery and an average final volume after the removing of water. The average final volume may range from about 10% to about 75% of the average synthesized volume such as, for example, about 10%, about 20%, about 30%, about 40%, about 50%, about 60%, about 70%, or about 75% of the average synthesized volume.

Lyophilization or freeze drying of the drug-loaded microbeads may be conducted according to any procedure commonly used for drying of particulate substances. For example, the drug-loaded microbeads may be placed in partially stoppered glass vials, which then are placed on a cooled, temperature-controlled shelf within a freeze dryer. The shelf temperature is reduced, and the sample is frozen to a uniform, defined temperature. After complete freezing, the pressure in the dryer may be decreased to a defined pressure to initiate primary drying. During the primary drying, water vapor is progressively removed from the frozen mass by sublimation while the shelf temperature is controlled at a constant, low temperature. Secondary drying may be initiated by increasing the shelf temperature and reducing the chamber pressure further so that water absorbed to the semi-dried mass can be removed until the residual water content decreases to the desired level.

Lyophilization or freeze drying of the drug-loaded microbeads may also be conducted by atmospheric pressure freeze drying by rapidly circulating very dry air over the frozen drug-loaded microbeads. The circulating dry gas provides improved heat and mass transfer from the frozen microbeads. Atmospheric spray drying processes may facilitate the formation of smalldiameter microbeads as a free-flowing powder, while avoiding formation of a dried cake. The free-flowing powder, in turn, may facilitate rehydration of the drug-loaded microbead composition when an embolization composition is prepared.

The drug-loaded microbeads of the drug-loaded microbead compositions previously described, or prepared according to the foregoing methods, may exhibit physical or mechanical properties beneficial to their use, storage, transport, or subsequent rehydration to form an embolization composition.

The drug-loaded microbeads of the drug-loaded microbead compositions previously described, or prepared according to the foregoing methods, may be provided to a physician with instructions for rehydrating the drug-loaded microbead composition to form an embolization composition that is ready for use in an embolization treatment. Accordingly, embodiments of this disclosure include embolization compositions and methods for preparing the embolization compositions.

An embolization composition may include a drug-loaded microbead composition according to any embodiment of this disclosure or a drug-loaded microbead composition prepared according to an embodied method of this disclosure. The embolization composition may further include amount of an aqueous solution sufficient to cause the drug-loaded microbeads of the drug-loaded microbead composition to reconstitute to a form suitable for administration to a patient. In particular, the amount of aqueous solution may cause the drug-loaded microbeads, after hydration, to have a water content of from about 50% by weight to about 99% by weight, from about 60% by weight to about 95% by weight, from about 70% by weight to about 95% by weight, from about 80% by weight to about 95% by weight, from about 90% by weight to about 95% by weight, or from about 80% by weight to about 99% by weight, or from about 90% by weight to about 99% by weight, or from about 95% by weight to about 99% by weight, based on the total weight of the drug-loaded microbeads. The aqueous solution may be any pharmaceutically acceptable solution such as, for, example, a physiologically buffered saline solution.

In example embodiments, the hydrated individual drug-loaded microbeads of the embolization composition include the biodegradable material, the therapeutic agent, the vesicular agents, and water. The hydrated individual drug-loaded microbeads of the embolization composition may include from about 3% by weight to about 10% by weight, from about 3% by weight to about 7% by weight, or from about 3.5% by weight to about 6.5% by weight, or from about 4% to about 6% by weight, or about 4.5% by weight to about 5.5% by weight, or about 5% to about 7% by weight biodegradable material, based on the total weight of the hydrated individual drug-loaded microbead. In example embodiments, the hydrated individual drug-loaded microbeads of the embolization composition may include from about 0.1% by weight to about 2.5% by weight, or from about 0.1% by weight to about 2% by weight, or from about 0.1% by weight to about 1.5% by weight, or from about 0.2% by weight to about 2.5% by weight, or from about 0.5% by weight to about 2.5% by weight, or from about 1% by weight to about 2.5% by weight therapeutic agent, based on the total weight of the hydrated individual drug-loaded microbead. In example embodiments, the hydrated individual drug-loaded microbeads of the embolization composition according to embodiments may have a water content of from about 50% by weight to about 99% by weight, from about 50% by weight to about 97% by weight, from about 50% by weight to about 96% by weight, from about 60% by weight to about 95% by weight, from about 70% by weight to about 95% by weight, from about 80% by weight to about 95% by weight, from about 90% by weight to about 95% by weight, or from about 80% by weight to about 99% by weight, or from about 90% by weight to about 99% by weight, or from about 95% by weight to about 99% by weight, based on the total weight of the individual drug-loaded microbeads.

The microbeads of the embolization composition may have average diameters from about 5 µm to about 1200 µm, as measured by dynamic light scattering (DLS). For nonspherical microbeads, the diameter of an individual particle is taken to be the widest measurement attainable from a first point on the surface of the microbead, through the center of mass of the microbead, to a second point on the surface of the microbead opposite the first point. The specific size distribution of the microparticles may be chosen or tailored to suit the particular treatment for which the embolization composition is intended to be used. The average diameters of the microbeads in the embolization composition may be chosen by any suitable method of size selection before or after the therapeutic agent is loaded into the microbeads. In some embodiments, the swollen microbeads of the embolization composition may have average diameters such as, for example, from about 40 µm to about 800 µm, from about 40 µm to about 100 µm, from about 40 µm to about 75 µm, from about 75 µm to about 100 µm, from about 100 µm to about 200 µm, from about 200 µm to about 300 µm, from about 300 µm to about 400 µm, from about 400 µm to about 500 µm, from about 600 µm to about 700 µm, or any subset of any of the foregoing ranges. In some embodiments, the swollen microbeads may have average diameters with a narrow size distribution such as 40 µm ± 20 µm, or 40 µm ± 10 µm, or 40 µm ± 5 µm, or 40 µm ± 1 µm, for example.

Methods for preparing an embolization composition may include adding a drug-loaded microbead composition according to any embodiment of this disclosure, or a drug-loaded microbead composition prepared according to an embodied method of this disclosure, to an amount of an aqueous solution sufficient to cause the drug-loaded microbeads of the drug-loaded microbead composition to swell, whereby the drug-loaded microbeads after swelling comprise a water content of from 50% to 99% by weight, from 50% to 90% by weight, from 50% to 75% by weight, from 60% to 99% by weight, from 75% to 99% by weight, from 75% to 95% by weight, from 75% to 90% by weight, or from 85% to 99% by weight, based on the total weight of the drug-loaded microbeads, to form an injection-ready solution. The aqueous solution may be any pharmaceutically acceptable solution such as, for, example, a physiologically buffered saline solution. The methods may further include loading the injection-ready solution into an injection device such as a syringe, for example. The methods may further include allowing the drug-loaded microbeads to swell for a rehydration time of from about 5 minutes to about 60 minutes before loading the injection-ready solution into the injection device. In some examples, the embolization composition may contain from about 25 mg therapeutic agent per milliliter of microbeads to about 150 mg therapeutic agent per milliliter of microbeads.

The embolization compositions prepared from the drug-loaded microbead compositions according to embodiments of this disclosure may be incorporated into an embolization treatment or therapy intended to treat a disease such as a cancer. In some embodiments, the therapeutic agent may be a chemotherapeutic agent having at least some efficacy for treating a cancer such as hepatocellular carcinoma, liver cancer, prostate cancer, or breast cancer. Accordingly, embodiments of this disclosure include methods for treating a disease. Methods for treating a disease may include providing intravenously, to a subject in need of embolization therapy, an embolization composition according to any embodiment previously described herein, prepared from a drug-loaded microbead composition according to any embodiment previously described herein.

In the methods for treating a disease, after the embolization composition is provided intravenously, at least a portion of the drug-loaded microbeads flow through vasculature of the subject to an embolization site and restrict blood flow through the embolization site. Thereafter, the drug-loaded microbeads may release to tissue at the embolization site over the course of a release period at least 90% by weight of the therapeutic agent, based on an initial amount of therapeutic agent embedded within the microbeads before the embolization composition is provided. In some embodiments, the release to the tissue at the embolization site may include an initial burst of drug release, during which at least 10% by weight of the therapeutic agent initially present in the drug-loaded microbeads is released into surrounding tissues at the embolization site. The initial burst may occur within 1 minute, 5 minutes, 10 minutes, 30 minutes, 60 minutes, 120 minutes, 180 minutes, 240 minutes, 300 minutes, 360 minutes, 12 hours, 18 hours, or 24 hours after the drug-loaded microbeads reach the embolization site. In some embodiments, the drug-loaded microbeads provide a sustained release of the therapeutic agent to the tissue over the release period. The release period may be an extended release period such as at least 5 days, at least 10 days, at least 14 days, at least 28 days, or at least 42 days. The end of release period is determined from the time when the drug-loaded microbeads are either completely decomposed or stop eluting therapeutic agent to the tissue.

Without intent to be bound by theory, it is believed that the release mechanism of the therapeutic agent from the drug-loaded microbeads of the embolization composition may be based on a dual mechanism including an ion exchange process and an enzymatic process. For example, it is believed that in an early stage of delivery of therapeutic agent to the embolization site, when plenty of water is present, the therapeutic agent may be released from the drug-loaded microbeads by ion exchange process. In a later stage, when water is lacking due to the vessel's being embolized, the drug-loaded microbeads may be degraded by an enzymatic process such as by lysozymes, for example, to release the therapeutic agent from the microbeads. It is believed that in the latest stage, such as by day 21 following implantation, most of the biodegradable material has been resorbed through tissue surrounding the embolization site. Because at this point the water-swellable material no longer has a matrix encapsulating the therapeutic agent or the complex of the therapeutic agent and the vesicular agents, the therapeutic agent can be completely eluted into the embolization site. Thereby, it is believed that the embolization compositions according to embodiments of this disclosure may increase tumor response and free survival rates. Additionally, the embolization compositions according to embodiments of this disclosure provide an economic value such as reducing the physician procedure time by eliminating a need to request a pharmacist or technician to add therapeutic agent to unloaded microbeads.

Further embodiments of this disclosure are directed to drug-loaded biodegradable microbead compositions. The drug-loaded biodegradable microbead compositions may include microbeads of a biodegradable material and at least one therapeutic agent embedded in a matrix of the biodegradable material, encapsulated by a matrix of the biodegradable material, disposed within a porous structure formed by the matrix of the biodegradable material, or ionically or non-covalently associated with a matrix of the biodegradable material. In such embodiments of drug-loaded biodegradable microbead compositions, the microbeads of the biodegradable material may contain, but need not necessarily contain, vesicular agents such as liposomes or ethosomes.

In the drug-loaded biodegradable microbead compositions, the biodegradable material is a biodegradable polymer or a lipid. Examples of biodegradable polymers include, without limitation, poly(lactide-co-glycolide) (PLGA), polylactide(PLA), polyglycolide (PGA), polycaprolactone (PCL), polyhydroxyalkanoates, poly-R-3-hydroxybutyrate (poly3HB), poly-R-3-hydroxybutyrate-co-R-3-hydroxyvalerate (poly(3HB-co-3HV)), poly-R-3-hydroxybutyrate-co-4-hydroxybutyrate (poly(3HB-co-4HB)), poly-R-3-hydroxyoctanoate-co-R-3-hydroxyhexanoate (poly(3HO-co-3HH)), poly-3-hydroxypropionate (poly(3HP)), poly-4-hydroxybutyrate (poly(4HB)), poly-5-hydroxybutyrate (poly(5HB)), poly-6-hydroxybutyrate (poly(6HB)), poly(propylene fumarate), poly(butylene succinate), poly(p-dioxanone, polyacetals, poly(ortho esters), polycarbonates, chitosan, hydroxybutyric acids, polyanhydrides and polyesters, polyphosphazenes, polyphosphoesters, lipodisq, celluloses, modified celluloses, proteins and poly(amino acids), polyethers, and co-polymers of the foregoing. Examples of lipids include, without limitation, tricaprin, trilaurin, trimyristin, tripalmitin, tristearin, hydrogenated coco-glycerides, glyceryl monostearate, glyceryl behenate, glyceryl palmitostearate, glyceryl caprate, cetyl palmitate, stearic acid, palmitic acid, decanoic acid, behenic acid, beeswax, carnauba wax, cacao butter, and combinations thereof. In example embodiments, the drug-loaded microbead compositions include individual microbeads are beads of a biodegradable cellulose or a biodegradable modified cellulose such as ethyl cellulose or cellulose acetate butyrate. The biodegradable polymer materials of the drug-loaded biodegradable microbead composition may include derivatives of any of the foregoing materials, or may include combinations of any of the foregoing materials or their derivatives. For example, the drug-loaded microbead compositions may include a combination of multiple biodegradable polymer materials, in which each individual microbead is made of a single type of polymer, and the drug-loaded biodegradable microbead composition includes microbeads of multiple polymer types. Alternatively, the drug-loaded biodegradable microbead compositions may include a combination of multiple biodegradable polymer materials, in which individual microbeads of the composition are composed of multiple types of polymer.

In various embodiments, the biodegradable material of the drug-loaded biodegradable microbead composition may include a solid lipid such as tricaprin, trilaurin, trimyristin, tripalmitin, tristearin, hydrogenated coco-glycerides, glyceryl monostearate, glyceryl behenate, glyceryl palmitostearate, glyceryl caprate, cetyl palmitate, stearic acid, palmitic acid, decanoic acid, behenic acid, beeswax, carnauba wax, and cacao butter. The biodegradable materials of the drug-loaded biodegradable microbead composition may include derivatives of any of the foregoing solid lipids, or may include combinations of any of the foregoing solid lipids or their derivatives. For example, the drug-loaded biodegradable microbead compositions may include a combination of multiple solid lipids, in which each individual microbead is made of a single type of solid lipid, and the drug-loaded biodegradable microbead composition includes microbeads of multiple solid lipids. Alternatively, the drug-loaded biodegradable microbead compositions may include a combination of multiple solid lipids, in which individual microbeads of the composition are composed of multiple types of solid lipids.

In the drug-loaded biodegradable microbead compositions, the at least one therapeutic agent may be chosen from doxorubicin, bevacizumab, bortezomib, imatinib, seliciclib, ceritinib, everolimus, paclitaxel, sorafenib, irinotecan, idarubicin, cisplatin, pharmaceutically acceptable salts and derivatives thereof, and combinations thereof. Further examples of specific therapeutic agents include, without limitation, doxorubicin, sorafenib, vandetanib, nivolumab, ipilimumab, regorafenib, irinotecan, epirubicin, pirarubicin, 5-fluorouracil, cisplatin, floxuridine, mitomycin C, derivatives of any of the foregoing, prodrugs of any of the foregoing, therapeutically acceptable salts or crystalline forms of any of the foregoing, or combinations of any of the foregoing. Further examples of suitable therapeutic agents include, without limitation, pirarubicin, mitoxantrone, tepotecan, paclitaxel, carboplatin, pemetrexed, penistatin, pertuzumab, trastuzumab, and docetaxel. Still further examples of suitable therapeutic agents include, without limitation, doxorubicin, bevacizumab, bortezomib, imatinib, seliciclib, ceritinib, everolimus, paclitaxel, sorafenib, irinotecan, idarubicin, cisplatin, and combinations of these drugs. In one specific embodiment of the drug-loaded biodegradable microbead compositions, the at least one therapeutic agent includes doxorubicin. In one specific embodiment of the drug-loaded biodegradable microbead compositions, the at least one therapeutic agent includes sorafenib. In one specific embodiment of the drug-loaded biodegradable microbead compositions, the at least one therapeutic agent includes paclitaxel. In some embodiments, the microbeads of the drug-loaded biodegradable microbead compositions include combinations of two, three, four, or greater than four therapeutic agents. In one specific embodiment of the drug-loaded biodegradable microbead compositions, the at least one therapeutic agent includes a combination of doxorubicin and sorafenib. In some embodiments, the biodegradable material and the at least one therapeutic agent are both hydrophobic, thereby facilitating homogeneous distribution of the therapeutic agent throughout the matrix of the biodegradable material.

The microbeads of the drug-loaded biodegradable microbead compositions may be prepared by any suitable process including, but not limited to, emulsion and microfluidics. Emulsion methods include preparing an aqueous phase of water and, optionally, an emulsifier such as polyvinyl alcohol; preparing an organic phase of a biodegradable material, a hydrophobic therapeutic agent, and a water-immiscible organic solvent; mixing the aqueous phase and the organic phase to form an emulsion; evaporating the solvents to dry the microbeads formed from the emulsion; recovering and further drying the microbeads; isolating desired size ranges of beads by filtering or sieving; optionally further drying and/or dehydrating and/or lyophilizing the microbeads; and sterilizing the microbeads. The microbeads may be rehydrated or reconstituted by adding a fluid such as water or physiologically buffered saline. Microbeads formed in this manned may have sizes from 20 µm to 900 µm, for example, and can be sieved to narrow ranges. The ratio of biodegradable material to therapeutic agent by weight may be from 100:1 to 0.01:1, or from 10:1 to 0.1:1, or any desired range between these ranges, for example. Microfluidics methods include similarly preparing aqueous and organic phases and, in a microfluidic apparatus, flowing small volumes of organic phase through the aqueous phase to produce small droplets in the aqueous phase that may be collected as microbeads at an exit of the apparatus.

Further embodiments of the present disclosure are directed to a drug-loaded microbead composition for use in a therapeutic embolization procedure. The drug-loaded microbead composition can be any of the drug-loaded microbead compositions that are described herein, and can be used in the form of an embolization composition according to any embodiment described herein. The therapeutic embolization procedure is preferably one for treating tumors. The therapeutic embolization procedure is preferably one for treating hepatocellular carcinoma, liver cancer, prostate cancer, or breast cancer.

Unless otherwise defined, all technical and scientific terms used herein have the same meaning as commonly understood by one of ordinary skill in the art to which the invention belongs. The terminology used in the description herein is for describing particular embodiments only and is not intended to be limiting. As used in the specification and appended claims, the singular forms "a," "an," and "the" are intended to include the plural forms as well, unless the context clearly indicates otherwise.

### EXAMPLES

The following examples are offered by way of illustration only. In view of the foregoing description, the person having ordinary skill in the art will recognize that the following examples are not intended to limit the scope of this disclosure or its many embodiments.

### Example 1

Liposomes including sorafenib incorporated within a lipid bilayer are prepared by subjecting a mixture of 37 mol.% poly(2-methacryloyloxyethyl phosphorylcholine) (PMPC), 12 mol.% dioleoyl phosphatidyl-serine (DOPS), 12 mol.% dioleoyl phosphatidyl-ethanolamine (DOPE), 26 mol% cholesterol and 13 Mol% sorafenib in chloroform solvent to a centrifugation extrusion with a 200-nm polycarbonate membrane. The size of the liposomes, as determined by Dynamic Light Scattering (DLS), is 171.8 ± 3.0 nm. Presence of sorafenib in the final liposome preparation is verified from an absorbance peak at about 270 nm, measured by UV-VIS spectroscopy.

### Example 2

Liposomes including doxorubicin surrounded by a lipid bilayer are prepared by subjecting a mixture of PMPC, DOPS, DOPE, and cholesterol in chloroform to a centrifugation extrusion with a 200-nm polycarbonate membrane. The chloroform solvent is dried from the mixture. The lipid mixture is then rehydrated with an aqueous solution of HEPES, salt, and doxorubicin, to form liposomes. Excess doxorubicin not incorporated within the liposomes is removed by gel filtration.

### Example 3

Ethosomes including sorafenib surrounded by a lipid bilayer are prepared by centrifugation extrusion with a 200-nm polycarbonate membrane of a mixture of 42 mol.% PMPC, 14 mol.% DOPS, 14 mol.% DOPE, and 30 mol.% cholesterol in a 40% ethanol/water solution containing 2 mM sorafenib. Free sorafenib is separated from the ethosomes by size exclusion chromatography on Sephadex G-25. The sorafenib ethosomes have sizes 234.4 ± 4.5 nm determined by dynamic light scattering. Presence of sorafenib in the final liposome preparation is verified from an absorbance peak at about 270 nm, measured by UV-VIS spectroscopy.

### Example 4

Liposomes including sorafenib within a lipid bilayer and doxorubicin surrounded by the lipid bilayer are prepared by subjecting a mixture of 37 mol.% poly(2-methacryloyloxyethyl phosphorylcholine) (PMPC), 12 mol.% dioleoyl phosphatidyl-serine (DOPS), 12 mol.% dioleoyl phosphatidyl-ethanolamine (DOPE), 26 mol.% cholesterol and 13 mol.% sorafenib in chloroform solvent to a centrifugation extrusion with a 200-nm polycarbonate membrane. The solvent is dried from the lipid/sorafenib mixture. The lipid mixture is then rehydrated with an aqueous solution of HEPES, salt, and doxorubicin, to form liposomes. Excess doxorubicin not incorporated within the liposomes is removed by gel filtration.

### Example 5

Vesicular agents such as the liposomes or ethosomes prepared according to any of Examples 1-4 are incorporated into microbeads of a biodegradable material by emulsion evaporation. An emulsion is prepared from an aqueous phase of deionized water and an emulsifier such as polyvinyl alcohol and an organic phase containing the vesicular agents and biodegradable polymer material.

An aqueous phase of 1% polyvinyl alcohol is prepared by stirring 8 g polyvinyl alcohol (86-89% hydrolyzed) into 800 mL deionized water at 600 rpm at 85 °C for 3-4 hours until the polyvinyl alcohol is fully dissolved.

An organic phase is prepared by adding to a 2-mL vial 150 mg cellulose acetate butyrate and 900 µL dichloromethane, sealing the vial, and shaking the vial until the cellulose acetate butyrate is dissolved. To the mixture, 100 µL of the vesicular agent in aqueous solution is added.

The organic phase and the aqueous phase are mixed by first adding to an appropriate vessel, such as a 250-mL flask, 100 mL of the aqueous phase then adding the contents of the vial of organic phase into the flask while stirring the aqueous phase in the flask at approximately 600 rpm. The flask is sealed with a lid, and stirring is continued for 30 minutes. After the thirty minutes, the lid is removed and the contents of the flask are stirred without the lid for an additional 2 hours. The stirring is then stopped, and the microbeads that have formed in the solution are allowed to settle.

The microbeads are then transferred by pipette to centrifuge tubes, and the tubes are centrifuged at 12000 rpm or greater for at least 2.5 minutes. The supernatant is then removed by transfer pipette, and the microbeads are washed by refilling the tubes a with deionized or filtered water and centrifuging again at 12000 rpm or greater for at least 2.5 minutes. The washing process is repeated from one to five times. The resulting microbeads have sizes from about 20 µm to about 350 µm.

The centrifuged microbeads are vacuum filtered through 200-micron mesh filters into a vacuum flask. The filtered microbeads in the flask have sizes from about 20 µm to about 200 µm. The filtered microbeads in the flask are then further vacuum filtered through a 100-micron mesh filter. The microbeads remaining on the filter are retained and have sizes from about 100 µm to about 200 µm. These microbeads are rinsed with filtered water then are allowed to dry in air for 24 hours. The air-dried microbeads then may be transferred to a suitable container or vial.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

## Claims

1. A drug-loaded microbead composition comprising:
a plurality of individual microbeads of a biodegradable material;
a plurality of individual vesicular agents contained within the individual microbeads or associated with the biodegradable material of the individual microbeads through ionic or non-covalent interaction, the vesicular agents comprising at least one lipid bilayer surrounding a vesicular core, wherein the vesicular agents comprise liposomes or ethosomes; and
a first therapeutic agent contained within the individual vesicular agents or associated with the individual vesicular agents by ionic or non-covalent interaction; and
a second therapeutic agent different from the first therapeutic agent and contained within the individual microbeads or associated with the individual microbeads through ionic or non-covalent interaction.

2. The drug-loaded microbead composition of claim 1, wherein the second therapeutic agent is contained within the individual vesicular agents or associated with individual vesicular agents through ionic or non-covalent interaction.

3. The drug-loaded microbead composition of any of the preceding claims, wherein the first therapeutic agent is hydrophilic and the second therapeutic agent is hydrophobic.

4. The drug-loaded microbead composition of claim 3, wherein the first therapeutic agent is encapsulated within the vesicular core of the individual vesicular agents and the second therapeutic agent is contained within the lipid bilayer of the individual vesicular agents.

5. The drug-loaded microbead composition of claim 3, wherein the second therapeutic agent is not contained within the individual vesicular agents and is associated by ionic or noncovalent interaction with the biodegradable material of the individual microbeads.

6. The drug-loaded microbead composition of any of the preceding claims, wherein the first therapeutic agent and the second therapeutic agent are independently chosen from doxorubicin, bevacizumab, bortezomib, imatinib, seliciclib, ceritinib, everolimus, paclitaxel, sorafenib, irinotecan, idarubicin, cisplatin, and combinations thereof.

7. The drug-loaded microbead composition of any of the preceding claims, wherein the biodegradable material is a biodegradable polymer chosen from poly(lactide-co-glycolide) (PLGA), polylactide(PLA), polyglycolide (PGA), polycaprolactone (PCL), polyhydroxyalkanoates, poly-R-3-hydroxybutyrate (poly3HB), poly-R-3-hydroxybutyrate-coR-3-hydroxyvalerate (poly(3HB-co-3HV)), poly-R-3-hydroxybutyrate-co-4-hydroxybutyrate (poly(3HB-co-4HB)), poly-R-3hydroxyoctanoate-co-R-3-hydroxyhexanoate (poly(3HO-co-3HH)), poly-3-hydroxypropionate (poly(3HP)), poly-4-hydroxybutyrate (poly(4HB)), poly-5-hydroxybutyrate (poly(5HB)), poly-6-hydroxybutyrate (poly(6HB)), poly(propylene fumarate), poly(butylene succinate), poly(p-dioxanone, polyacetals, poly(ortho esters), polycarbonates, chitosan, hydroxybutyric acids, polyanhydrides and polyesters, polyphosphazenes, polyphosphoesters, lipodisq, celluloses, modified celluloses, proteins and poly(amino acids), polyethers, and co-polymers of the foregoing.

8. The drug-loaded microbead composition of any of the preceding claims, wherein the biodegradable material is a cellulose.

9. The drug-loaded microbead composition of any of the preceding claims, wherein the biodegradable material is a modified cellulose comprising cellulose acetate butryrate.

10. The drug-loaded microbead composition of any of claims 1 to 6, wherein the biodegradable material is a lipid chosen from tricaprin, trilaurin, trimyristin, tripalmitin, tristearin, hydrogenated coco-glycerides, glyceryl monostearate, glyceryl behenate, glyceryl palmitostearate, glyceryl caprate, cetyl palmitate, stearic acid, palmitic acid, decanoic acid, behenic acid, beeswax, carnauba wax, cacao butter, and combinations thereof.

11. The drug-loaded microbead composition of claim 1, wherein:
the individual vesicular agents comprise liposomes;
the first therapeutic agent is contained within the lipid bilayer of the individual vesicular agents;
the second therapeutic agent is not contained within the individual vesicular agents and is associated by ionic or non-covalent interaction with the biodegradable material of the individual microbeads; and
the biodegradable material is a cellulose.

12. The drug-loaded microbead composition of claim 1, wherein:
the individual vesicular agents comprise liposomes;
the first therapeutic agent is contained within the lipid bilayer of the individual vesicular agents;
the second therapeutic agent is encapsulated within the vesicular core of the individual vesicular agents;
the first therapeutic agent is sorafenib;
the second therapeutic agent is doxorubicin; and
the biodegradable material is a cellulose.

13. The drug-loaded microbead composition of claim 1, wherein:
the individual vesicular agents comprise ethosomes;
the first therapeutic agent is encapsulated within the vesicular core of the individual vesicular agents;
the second therapeutic agent is not contained within the individual vesicular agents and is associated by ionic or non-covalent interaction with the biodegradable material;
the first therapeutic agent is sorafenib;
the second therapeutic agent is doxorubicin; and
the biodegradable material is a cellulose.

14. The drug-loaded microbead composition of any of the preceding claims, wherein the at least one lipid bilayer comprises poly(2-methacryloyloxyethyl phosphorylcholine) (PMPC), dioleoyl phosphatidylserine (DOPS), dioleoyl phosphatidylethanolamine (DOPE), and cholesterol.

## Patentansprüche

1. Arzneimittelbeladene Mikrokugel-Zusammensetzung, umfassend:
eine Vielzahl von individuellen Mikrokugeln aus einem biologisch abbaubaren Material;
eine Vielzahl von individuellen vesikulären Agenzien, die in den individuellen Mikrokugeln enthalten sind oder mit dem biologisch abbaubaren Material der individuellen Mikrokugeln durch eine ionische oder nicht-kovalente Wechselwirkung assoziiert sind, wobei die vesikulären Agenzien mindestens eine Lipid-Doppelschicht umfassen, die einen vesikulären Kern umgibt, wobei die vesikulären Agenzien Liposomen oder Ethosomen umfassen; und
ein erstes therapeutisches Agens, das in den individuellen vesikulären Agenzien enthalten ist oder mit den individuellen vesikulären Agenzien durch eine ionische oder nicht-kovalente Wechselwirkung assoziiert ist; und
ein zweites therapeutisches Agens, das von dem ersten therapeutischen Agens unterschiedlich ist und in den individuellen Mikrokugeln enthalten ist oder mit den individuellen Mikrokugeln durch eine ionische oder nicht-kovalente Wechselwirkung assoziiert ist.

2. Arzneimittelbeladene Mikrokugel-Zusammensetzung nach Anspruch 1, wobei das zweite therapeutische Agens in den individuellen vesikulären Agenzien enthalten ist oder mit den individuellen vesikulären Agenzien durch eine ionische oder nicht-kovalente Wechselwirkung assoziiert ist.

3. Arzneimittelbeladene Mikrokugel-Zusammensetzung nach einem der vorstehenden Ansprüche, wobei das erste therapeutische Agens hydrophil ist und das zweite therapeutische Agens hydrophob ist.

4. Arzneimittelbeladene Mikrokugel-Zusammensetzung nach Anspruch 3, wobei das erste therapeutische Agens in dem vesikulären Kern der individuellen vesikulären Agenzien verkapselt ist und das zweite therapeutische Agens in der Lipid-Doppelschicht der individuellen vesikulären Agenzien enthalten ist.

5. Arzneimittelbeladene Mikrokugel-Zusammensetzung nach Anspruch 3, wobei das zweite therapeutische Agens nicht in den individuellen vesikulären Agenzien enthalten ist und durch eine ionische oder nicht-kovalente Wechselwirkung mit dem biologisch abbaubaren Material der individuellen Mikrokugeln assoziiert ist.

6. Arzneimittelbeladene Mikrokugel-Zusammensetzung nach einem der vorstehenden Ansprüche, wobei das erste therapeutische Agens und das zweite therapeutische Agens unabhängig ausgewählt sind aus Doxorubicin, Bevacizumab, Bortezomib, Imatinib, Seliciclib, Ceritinib, Everolimus, Paclitaxel, Sorafenib, Irinotecan, Idarubicin, Cisplatin und Kombinationen davon.

7. Arzneimittelbeladene Mikrokugel-Zusammensetzung nach einem der vorstehenden Ansprüche, wobei das biologisch abbaubare Material ein biologisch abbaubares Polymer ist, ausgewählt aus Poly(lactid-co-glycolid) (PLGA), Polylactid (PLA), Polyglycolid (PGA), Polycaprolacton (PCL), Polyhydroxyalkanoaten, Poly-R-3-hydroxybutyrat (poly3HB), Poly-R-3-hydroxybutyrat-co-R-3-hydroxyvalerat (poly(3HB-co-3HV)), Poly-R-3-hydroxybutyrat-co-4-hydroxybutyrat (poly(3H B-co-4H B)), Poly-R-3-hydroxyoctanoat-co-R-3-hydroxyhexanoat (poly(3HO-co-3HH)), Poly-3-hydroxypropionat (poly(3HP)), Poly-4-hydroxybutyrat (poly(4HB)), Poly-5-hydroxybutyrat (poly(5HB)), Poly-6-hydroxybutyrat (poly(6HB)), Poly(propylenfumarat), Poly(butylensuccinat), Poly(p-dioxanon), Polyacetalen, Poly(orthoestern), Polycarbonaten, Chitosan, Hydroxybuttersäuren, Polyanhydriden und Polyestern, Polyphosphazenen, Polyphosphoestern, Lipodisq, Cellulosen, modifizierten Cellulosen, Proteinen und Poly(aminosäuren), Polyethern und Co-Polymeren der zuvor genannten.

8. Arzneimittelbeladene Mikrokugel-Zusammensetzung nach einem der vorstehenden Ansprüche, wobei das biologisch abbaubare Material eine Cellulose ist.

9. Arzneimittelbeladene Mikrokugel-Zusammensetzung nach einem der vorstehenden Ansprüche, wobei das biologisch abbaubare Material eine modifizierte Cellulose ist, die Celluloseacetatbutyrat umfasst.

10. Arzneimittelbeladene Mikrokugel-Zusammensetzung nach einem der Ansprüche 1 bis 6, wobei das biologisch abbaubare Material ein Lipid ist, ausgewählt aus Tricaprin, Trilaurin, Trimyristin, Tripalmitin, Tristearin, hydrogenierten Cocoglyceriden, Glycerylmonostearat, Glycerylbehenat, Glycerylpalmitostearat, Glycerylcaprat, Cetylpalmitat, Stearinsäure, Palmitinsäure, Decansäure, Behensäure, Bienenwachs, Carnaubawachs, Kakaobutter und Kombinationen davon.

11. Arzneimittelbeladene Mikrokugel-Zusammensetzung nach Anspruch 1, wobei:
die individuellen vesikulären Agenzien Liposomen umfassen;
das erste therapeutische Agens in der Lipid-Doppelschicht der individuellen vesikulären Agenzien enthalten ist;
das zweite therapeutische Agens nicht in den individuellen vesikulären Agenzien enthalten ist und durch eine ionische oder nicht-kovalente Wechselwirkung mit dem biologisch abbaubaren Material der individuellen Mikrokugeln assoziiert ist; und
das biologisch abbaubare Material eine Cellulose ist.

12. Arzneimittelbeladene Mikrokugel-Zusammensetzung nach Anspruch 1, wobei:
die individuellen vesikulären Agenzien Liposomen umfassen;
das erste therapeutische Agens in der Lipid-Doppelschicht der individuellen vesikulären Agenzien enthalten ist;
das zweite therapeutische Agens in dem vesikulären Kern der individuellen vesikulären Agenzien verkapselt ist;
das erste therapeutische Agens Sorafenib ist;
das zweite therapeutische Agens Doxorubicin ist; und
das biologisch abbaubare Material eine Cellulose ist.

13. Arzneimittelbeladene Mikrokugel-Zusammensetzung nach Anspruch 1, wobei:
die individuellen vesikulären Agenzien Ethosomen umfassen;
das erste therapeutische Agens in dem vesikulären Kern der individuellen vesikulären Agenzien verkapselt ist;
das zweite therapeutische Agens nicht in den individuellen vesikulären Agenzien enthalten ist und durch eine ionische oder nicht-kovalente Wechselwirkung mit dem biologisch abbaubaren Material assoziiert ist;
das erste therapeutische Agens Sorafenib ist;
das zweite therapeutische Agens Doxorubicin ist; und
das biologisch abbaubare Material eine Cellulose ist.

14. Arzneimittelbeladene Mikrokugel-Zusammensetzung nach einem der vorstehenden Ansprüche, wobei die mindestens eine Lipid-Doppelschicht Poly(2-methacryloyloxyethylphosphorylcholin) (PMPC), Dioleoylphosphatidylserin (DOPS), Dioleoylphosphatidylethanolamin (DOPE) und Cholesterin umfasst.

## Revendications

1. Composition de microbilles chargées de médicament comprenant :
une pluralité de microbilles individuelles d'un matériau biodégradable ;
une pluralité d'agents vésiculaires individuels contenus à l'intérieur des microbilles individuelles ou associés au matériau biodégradable des microbilles individuelles à travers une interaction ionique ou non covalente, les agents vésiculaires comprenant au moins une bicouche lipidique entourant un noyau vésiculaire, dans laquelle les agents vésiculaires comprennent des liposomes ou des éthosomes ; et
un premier agent thérapeutique contenu à l'intérieur des agents vésiculaires individuels ou associé aux agents vésiculaires individuels par une interaction ionique ou non covalente ; et
un second agent thérapeutique différent du premier agent thérapeutique et contenu à l'intérieur des microbilles individuelles ou associé aux microbilles individuelles à travers une interaction ionique ou non covalente.

2. Composition de microbilles chargées de médicament selon la revendication 1, dans laquelle le second agent thérapeutique est contenu à l'intérieur des agents vésiculaires individuels ou associé à des agents vésiculaires individuels à travers une interaction ionique ou non covalente.

3. Composition de microbilles chargées de médicament selon l'une quelconque des revendications précédentes, dans laquelle le premier agent thérapeutique est hydrophile et le second agent thérapeutique est hydrophobe.

4. Composition de microbilles chargées de médicament selon la revendication 3, dans laquelle le premier agent thérapeutique est encapsulé à l'intérieur du noyau vésiculaire des agents vésiculaires individuels et le second agent thérapeutique est contenu à l'intérieur de la bicouche lipidique des agents vésiculaires individuels.

5. Composition de microbilles chargées de médicament selon la revendication 3, dans laquelle le second agent thérapeutique n'est pas contenu à l'intérieur des agents vésiculaires individuels et est associé par une interaction ionique ou non covalente au matériau biodégradable des microbilles individuelles.

6. Composition de microbilles chargées de médicament selon l'une quelconque des revendications précédentes, dans laquelle le premier agent thérapeutique et le second agent thérapeutique sont indépendamment choisis parmi la doxorubicine, le bévacizumab, le bortézomib, l'imatinib, le seliciclib, le céritinib, l'évérolimus, le paclitaxel, le sorafénib, l'irinotécan, l'idarubicine, la cisplatine, et des combinaisons de ceux-ci.

7. Composition de microbilles chargées de médicament selon l'une quelconque des revendications précédentes, dans laquelle le matériau biodégradable est un polymère biodégradable choisi parmi poly(lactide-co-glycolide) (PLGA), polylactide (PLA), polyglycolide (PGA), polycaprolactone (PCL), polyhydroxyalcanoates, poly-R-3-hydroxybutyrate (poly(3HB)), poly- R-3-hydroxybutyrate-co-R-3-hydroxyvalérate (poly(3HB-co-3HV)), poly-R-3-hydroxybutyrate-co-4-hydroxybutyrate (poly(3HB-co-4HB)), poly-R-3-hydroxyoctanoate-co-R-3-hydroxyhexanoate (poly(3HO-co-3HH)), poly-3-hydroxypropionate (poly(3HP)), poly-4-hydroxybutyrate (poly(4HB)), poly-5-hydroxybutyrate (poly(5HB)), poly-6-hydroxybutyrate (poly(6HB)), poly(propylène fumarate), poly(butylène succinate), poly(p-dioxanone), polyacétals, poly(ortho esters), polycarbonates, chitosane, acides hydroxybutyriques, polyanhydrures et polyesters, polyphosphazènes, polyphosphoesters, lipodisq, celluloses, celluloses modifiées, protéines et poly(aminoacides), polyéthers, et co-polymères des éléments précités.

8. Composition de microbilles chargées de médicament selon l'une quelconque des revendications précédentes, dans laquelle le matériau biodégradable est une cellulose.

9. Composition de microbilles chargées de médicament selon l'une quelconque des revendications précédentes, dans laquelle le matériau biodégradable est une cellulose modifiée comprenant de l'acétate butyrate de cellulose.

10. Composition de microbilles chargées de médicament selon l'une quelconque des revendications 1 à 6, dans laquelle le matériau biodégradable est un lipide choisi parmi la tricaprine, la trilaurine, la trimyristine, la tripalmitine, la tristéarine, des cocoglycérides hydrogénés, le monostéarate de le glycéryle, le béhénate de glycéryle, le palmitostéarate de glycéryle, le caprate de glycéryle, le palmitate de cétyle, l'acide stéarique, l'acide palmitique, l'acide décanoïque, l'acide béhénique, la cire d'abeille, la cire de carnauba, le beurre de cacao, et des combinaisons de ceux-ci.

11. Composition de microbilles chargées de médicament selon la revendication 1, dans laquelle :
les agents vésiculaires individuels comprennent des liposomes ;
le premier agent thérapeutique est contenu à l'intérieur de la bicouche lipidique des agents vésiculaires individuels ;
le second agent thérapeutique n'est pas contenu à l'intérieur des agents vésiculaires individuels et est associé par une interaction ionique ou non covalente au matériau biodégradable des microbilles individuelles ; et le matériau biodégradable est une cellulose.

12. Composition de microbilles chargées de médicament selon la revendication 1, dans laquelle :
les agents vésiculaires individuels comprennent des liposomes ;
le premier agent thérapeutique est contenu à l'intérieur de la bicouche lipidique des agents vésiculaires individuels ;
le second agent thérapeutique est encapsulé à l'intérieur du noyau vésiculaire des agents vésiculaires individuels ;
le premier agent thérapeutique est le sorafénib ;
le second agent thérapeutique est la doxorubicine ; et
le matériau biodégradable est une cellulose.

13. Composition de microbilles chargées de médicament selon la revendication 1, dans laquelle :
les agents vésiculaires individuels comprennent des éthosomes ;
le premier agent thérapeutique est encapsulé à l'intérieur du noyau vésiculaire des agents vésiculaires individuels ;
le second agent thérapeutique n'est pas contenu à l'intérieur des agents vésiculaires individuels et est associé par une interaction ionique ou non covalente au matériau biodégradable ;
le premier agent thérapeutique est le sorafénib ;
le second agent thérapeutique est la doxorubicine ; et
le matériau biodégradable est une cellulose.

14. Composition de microbilles chargées de médicament selon l'une quelconque des revendications précédentes, dans laquelle l'au moins une bicouche lipidique comprend de la poly(2-méthacryloyloxyéthyl phosphorylcholine) (PMPC), de la di-oléoyl phosphatidylsérine (DOPS), de la di-oléoyl phosphatidyléthanolamine (DOPE), et du cholestérol.
